# EUROPEAN PATENT APPLICATION

(11) **EP 1 695 715 A1**
(43) Date of publication of application: **30.08.2006**
(21) Application number: 04802606.6
(22) Date of filing: 10.11.2004
(51) Int. Cl.: A61K 39/002, C12N 15/30, C07K 14/44, A61P 33/04

(54) **VACCINE COMPOSITIONS WHICH ARE USED TO CONTROL AMOEBIASIS AND WHICH ARE BASED ON THE 112KDA SURFACE PROTEIN FROM ENTAMOEBA HISTOLYTICA**

(30) Priority: 11.11.2003 CU 25903
(71) Applicant: Instituto Finlay, Centro de Investigacion-Produccion de vacunas y sueros, La Coronela, La Lisa, Ciudad de la Habana 12100 (CU); Centro de Investigacion y de Estudios Avanzados del Instituto Politécnico Nacional, México D.F., 07380 (MX)
(72) Inventor: OROZCO OROZCO, Maria E., Via Ceti No. 20., México D.F., México CP: 07730 (MX); SIERRA GONZALEZ, Gustavo Victoriano, Ciudad de la Habana 11600 (CU); GARCIA MADRIZ, Xochil, Calle 15 No. 1306, Vedado, Ciudad de la Habana 10400 (CU); MARTINEZ BENITEZ, Máximo B., 5ta Ave., No. 18, CP: 11600, C.Habana, Ciudad de la Habana (CU); RODRIGUEZ RODRIGUEZ, Mario A., Deutz Hermanos, 31, Estado de México, Tlanepantla, 54080 (MX); GARCIA RIVERA, G., Plazuela 2 de Plaza Sésamo, Nezahulcoyolt, CP: 57139 (MX); ACOSTA DOMINGUEZ, Armando, Ciudad de la Habana 11300 (CU)
(74) Representative: van Loon, C.J.J.
(86) International application number: PCT/CU2004/000013
(87) International publication number: WO 2005/044300

(57) **Abstract**

SUMMARY

This invention relates to the field of immunology, specifically to vaccinal compositions for the control of infections caused by *Entamoeba histolytica.*

The active principle of the compositions of the invention is a 112kDa amoebian surface protein, coded by two genes, one coding for a peptide with cystein protease activity and the other for a peptide with adhesin activity. The scope of the invention includes formulations containing the genes coding for said protein, which are administered as DNA vaccines and also formulations containing the purified proteins or fragments thereof, achieving a synergic effect as regards protection with the combination.

These formulations can be administered for prophylactic or therapeutical purposes to fight against the amoebian infection caused by the protozoan parasite *Entamoeba histolytica.*

## Description

### Field of the Invention

This invention relates to the field of immunology, specifically to the development of control methods for infections by *Entamoeba histolytica,* based on vaccines.

### Prior Art

*Entamoeba histolytica,* a protozoan widely distributed throughout the world, is responsible for human amebiasis. It is calculated that 10% of the world's population is carrier of the disease, even though less than 1 % of such carriers develop the same (Walsh, J.A. Prevalence of *Entamoeba histolytica* infection. In: J. Ravdin, Ed. Amebiasis: human infection by *Entamoeba Histolytica.* New York: Wiley Medical, 1988; 93-105).

An estimate made in 1984 indicated that 500 million people were infected and among them, 40 million developed disabling colitis or extraintestinal abscesses. The number of deaths due to this cause was 40,000 that year (Walsh, J.A. Prevalence of *Entamoeba histolytica* infection. In: J. Ravdin, Ed. Amebiasis: human infection by *Entamoeba Histolytica* New York: Wiley Medical, 1988; 93-105). In a WHO bulletin issued 9 years later, it was informed that this microorganism is responsible for up to 100 thousand deaths per annum, placing it second only to malaria in mortality due to protozoan parasites (WHO. Amoebiasis. *WHO Weekly Epidemiology Record* 1997; 72: 97-100.). This evidences, on the one hand, the increasingly serious health problem posed by amebiasis for human kind and on the other, the insufficiency of measures to control the same.

The geographical distribution of this parasitosis is not exclusive of tropical regions; it has been found in all climates, including sub-polar regions (Salata, R.A., Ravdin, J.I. Immunoprophylaxis. In: J. Ravdin, Ed. Amebiasis: human infection by *Entamoeba Histolytica.* New York: Wiley Medical, 1988; 785-801). However, due to the faecal-oral transmission route of the amoeba, the areas with highest incidence rates are those with the lowest socio-economic development. The absence of sanitary measures, misery and ignorance can account for the endemic behaviour of this disease. (Caballero Salcedo A., Viveros Rogel M, Salvatierra B, Tapia Conyer R, Sepúlveda Amor J, Gutierrez G and Ortiz Ortiz L. Seroepidemiology of Amebiasis in Mexico. Am J Trop Med Hyg 1994; 50: 412-419). For example, in Mexico, amoebiasis is a major national public health problem. In a survey carried out in 1994, it was found that 8.4% of the population was seropositive to *Entamoeba histolytica* antigens. (Caballero Salcedo A., Viveros Rogel M, Salvatierra B, Tapia Conyer R, Sepúlveda Amor J, Gutierrez G and Ortiz Ortiz L. Seroepidemiology of Amebiasis in Mexico. Am J Trop Med Hyg 1994; 50: 412-419). In Cuba, this disease does not pose a major health problem and it can be mentioned a study carried out in a Primary School of Havana City, wherein 425 children were analysed and among them only 5 (1.1%) showed infection by *Entamoeba histolytica* (Arencibia A. et al. Parásitos intestinales en niños que asisten a una Escuela Primaria Urbana de Ciudad de la Habana. Boletin Epidemiológico Semanal del IPK. 2001; vol. 11 No. 8.). In the year 2000, an accumulated total for the whole country of 8 cases of acute amoebic dysentery was reported, which amounted to a rate of 0.68 per 100,000 inhabitants (Tables: Enfermedades de Declaración Obligatorias (EDO) Seleccionadas (Boletin Epidemiológico Semanal del IPK. 2001; vol. 11 No. 8.)).

The pathogenic mechanism of the amoeba is a complex and multifactorial process, which has not yet been fully understood. In spite of this, it is the consensus among experts studying this subject that at least 3 events are essential for colonization of intestinal epithelium: (Petri WA, Ravdin JI. In vitro models of amebic pathogenesis. In: J. Ravdin, Ed. Amebiasis: human infection by *Entamoeba Histolytica.* New York: Wiley Medical, 1988; 191-204).
1. Adherence of trophozoites to target cell.
2. Cytopathic effect is contact-dependent.
3. Phagocytosis.

Adherence is an important virulence factor for pathogen microorganisms, allowing establishment, reproduction and colonization of host tissues (Ravdin JI. Pathogenesis of Amebiasis: An overview. In: J. Ravdin, Ed. Amebiasis: human infection by *Entamoeba Histolytica.* New York: Wiley Medical, 1988; 166-176).

Pathogen molecules specialized in establishing these specific attachments to host cells are known as adhesins and some of them have been characterized. Among them, we find the following:

The 260 kDa lectin. Adherence to human colon mucins and epithelial cells is mediated by this lectin (Ravdin JI, John JE, Johnston LI, Innes DJ, Guerrant RL. Adherence of trophozoites to rat and human colonic mucosa. Infect Immun 1985; 48: 292-297). Currently there are monoclonal antibodies directed against the amoeba and capable of blocking the adherence that recognizes purified lectin (Petri WA Jr, Smith RD, Schlesinger PH, Murphy CF, Ravdin JI. Isolation of the galactose binding lectin of Entamoeba histolytica J.Clin. Invest 1987; 80: 1238-1244). The 220 kDa lectin is a membrane protein capable of binding to MDCK cells (a cell line isolated from dog kidney (Madin Darby canine kidney cell)) and of hemagglutinating human erythrocytes.

The 112 kDa adhesin was identified using adherence-deficient mutants and monoclonal antibodies. These antibodies inhibit trophozoites adherence to erythrocytes and epithelial cells. This protein is affected in adherence-deficient mutants (Arroyo R, Orozco E. Localization and identification of an Entamoeba histolytica adhesin. Mol Biochem Parasitol 1987; 23: 151-158).

Recently it has been found that it is an heterodimer constituted by a 78 kDa polypeptide with adhesin function and another 49 kDa polypeptide with cystein-protease activity (Garcia Rivera G, Rodriguez MA, Ocadiz R, Martinez-Lopez C, Arroyo R, Gonzalez Robles A, Orozco E. *Entamoeba histolytica:* a novel cystein protease and an adhesin form the 112 kDa surface protein. Molecular Microbiology 1999; 33: 556-568). It is also known that monoclonal antibodies directed against the 150 kDa lectin, a surface glycoprotein, are capable of inhibiting the adherence of amoebas to erythrocytes and CHO cells, as well as the erythrophagocytosis and cytotoxicity in CHO cells: (Cheng XJ, Tusukamoto H, Kaneda Y, Tachibana H. Identification of the 150 kDa surface antigen of *Entamoeba histolytica* as a galactose and N- acetyl-D galactosamine-inhibitable lectin. Parasitol Res 1997; 84:632-639).

E. histolytica is one of the most potent cytotoxic cells known. Its name is due precisely to its incredible capacity to destroy human tissues. It disrupts the intestinal barrier secreting enzymes and lysing host epithelial and inflammatory cells (Ravdin JI. *Entamoeba histolytic*a: from Adherence to Enteropathy. J. Infect. Dis 1989; 159: 420-429).

The intestinal mucosa is the most important barrier against enteric microorganisms and secretory IgA antibodies play an important role in the exclusion of pathogens from the epithelial surface. Type A immunoglobulins against *E. histolytica* have been detected in biliary secretions, human milk, calostrum, saliva and intestinal fluids (Ortiz Ortiz L, Mora N, Zambrano Villa SG, Carrero JC, Sanchez Zerpa M, Osuna A, Rosales Borges DM. Secretory immune response in patients with intestinal amoebiasis. Parasite immunol 1998; 20: 503-507). Nevertheless, their role in immune protection against amoebian infection is still under investigation.

The efforts carried out so far have proved fruitless in practice since *E. histolytica* is capable of evading antibodies immobilization (Calderon J, Muñoz ML, Acosta HM. Surface redistribution and release of antibody-induced caps in Entamoeba. *J Exp Med* 1980; 151: 184-193), the classic complement-mediated lysis; important aspects in the protective immune response are unknown, such as the role of immune system cells in such response (Ravi VV, Mithal S, Malavinya AN, Tandon BN. Immunologic studies in amebic liver abscess. Trans R Soc Trop Med Hyg 1980; 74: 300), the plasticity of amoebian DNA in gene expression and host factors not well studied have also influenced the same.

On the other hand, studies carried out in animal models and clinic studies have contributed evidences of the central role played by cell-mediated immune mechanisms in host defence against the amoeba. (Salata RA, Martinez Palomo A, Murphy CF, Canales L, Segovia E, Trevino N. Murray HW, Ravdin JI. Patients treated for amebic liver abscess develop cell-mediated immune responses effective in vitro against *Entamoeba histolytica.* J Immunol 1986b; 136 :2633-2639; Schain DC, Salata RA, Ravdin JI. Development of amebicidal cell mediated immunity in gerbils (Meriones unguiculatus) immunized with the galactose inhibitable adherence lectin of *Entamoeba histolytica. J Parasitol* 1995; 81: 563- 568; Ghost PK, Castellanos Barba C, Ortiz Ortiz L. Intestinal amebiasis: cyclic suppression of the immune response. *Parasitol Res* 1995; 81: 205-210; Alba-Hurtado F, Diaz Otero F, Valdivia G, Reyes R, Tsutsumi V, Acosta G. Cellular immune response of intracecal inoculated Mongolian gerbils with *Entamoeba histolytica:* trophozoites. Rev latinoam Microbiol 1999; 41: 205-210). There are evidences proving that T-lymphocytes from patients cured from amoebian liver abscesses present a specific proliferative response to *E. histolytica* antigens and production of limphokynes (IL-2 and INF) (Salata RA, Pearson RP, Murphy CF, Ravdin JI. Interaction of human leukocytes with *Entamoeba histolytica:* killing of virulent amebae by the activated macrophage: *J.Clin. Invest* 1985a; 76:491) and that at least in vitro, they induce an amoebicidal activity in monocyte-derived macrophages (Salata RA, Martinez Palomo A, Murphy CF, Canales L, Segovia E, Trevino N. Murray HW, Ravdin JI. Patients treated for amebic liver abscess develop cell-mediated immune responses effective in vitro against *Entamoeba histolytica.* J Immunol 1986b; 136 :2633-2639; Schain DC, Salata RA, Ravdin JI. Human T lymphocyte proliferation, lymphokine production, and amebicidal activity elicited by the galactose inhibitable adherence protein of Entamoeba histolytica. Infect Immun 1992; 60:2143- 2146).

However, resistance mechanisms of activated macrophages and stimulated T-lymphocytes against the cytolytic power of the amoebas have not been determined.

It is important to note that the activation of an inappropriate response may aggravate the disease (Mosmann TR, Sad S. The expanding universe of T-cell subsets: Th1, Th2 and more. Immunol Today 1996; 17: 138-146). The production of IL-4, IL-10 and TGF (response Th2) may inhibit the immune response to *E. histolytica* (Lin JY, Chadee K. Macrophage cytotoxicity against *Entamoeba histolytica* trophozoites is mediated by nitric oxide from L-arginine. *J Immunol* 1992; 148: 3999-4005). PGE2 produced at an early stage of the infection by amoebas (Wang W, Chadee K. *Entamoeba histolytica* alters arachidonic acid metabolism in macrophages in vitro and in vivo. Immunology 1992; 76: 242-250), stimulates IL-4 transcription and depresses that of IL-2, which may account for the transient immunosuppression observed at this phase. (Lacour M, Arrighi JF, Muller KM, Carlberg C, Sauret JH, Hauser C. cAMP up-regulates IL-4 and IL-5 production from activated CD4+ T cell while decreasing IL-2 release and NF-AT induction. *Int. Immunol* 1994; 6:1333-1343)
Taking into account that humans are the only relevant host for E. histolytica, it has been suggested that an effective vaccination program might eradicate amoebiasis (Stanley SL.

Progress towards an amebiasis *vaccine. Parasitology Today.* 1996; 12: 7-14). This is why parasite proteins playing crucial roles in the pathogenesis of the disease, especially those involved in adherence (Kobiler D, Mirelman D. Lectin activity in *Entamoeba histolytica* trophozoites. *Infect. Immun* 1980; 29: 221-225; Zhang T, Cieslak PR, Stanley SL. Protection of gerbils from amebic liver abscess by immunization with a recombinant *Entamoeba histolytica* antigen. *Infect Immun* 1994a; 62: 1166-1170; Dodson MJ, Lenkowski PJ Jr, Eubanks AC, Jackson TFGH, Napodano J, Leyerly DM, Lockhart LA, Mann BJ, Petri WA. Infection and

Immunity mediated by the carbohydrate recognition domain of the *Entamoeba histolytica* Gal-GalNac lectin. *J Infect Dis* 1999; 179: 460-466), have given rise to additional interest for their use as vaccinal candidates.

One of the surface molecules better characterized for this purpose is the galactose-specific and N-acetyl D galactosamine lectin heterodimer (260 kDa). The 170 kDa subunit has turned to be the most interesting from the immunologic point of view, since protection in animal models has been reported after the administration of the natural, recombinant protein, of peptides derived from the same and of DNA vaccines by different routes (Lotter H, Khajawa F; Stanley SL Jr; Tannich E. Protection of gerbils from amebic liver abscess by vaccination with a 25-mer peptide derived from the cystein-rich region of *Entamoeba histolytica* galactose-specific adherence lectin. *Infect. Immun* 2000, 68:4416-4421), (Kelsall BL, Ravdin, J.I. Immunization of rats with 260-kilodalton *Entamoeba histolytica* galactose inhibitable lectin elicits an intestinal secretory immunoglobulin A response that has in vitro adherence inhibitory activity. *Infect Immun 1995;* 63: 686-689), (Dodson MJ, Lenkowski PJ Jr, Eubanks AC, Jackson TFGH, Napodano J, Leyerly DM, Lockhart LA, Mann BJ, Petri WA. Infection and Immunity mediated by the carbohydrate recognition domain of the *Entamoeba histolytica* Gal-GalNac lectin. *J Infect Dis* 1999; 179: 460-466), (Lotter H, Zhang T, Seydel KB, Stanley SL Jr; Tannich E. Identification of an epitope on the *Entamoeba histolytica* 170 kDa lectin conferring antibody-mediated protection against invasive amebiasis. *J Exp Med* 1997; 185: 1793-1803), (Zhang T, Cieslak PR, Stanley SL. Protection of gerbils from amebic liver abscess by immunization with a recombinant *Entamoeba histolytica* antigen. *Infect Immun* 1994a; 62: 1166-1170), (Mann BJ et al. Neutralizing monoclonal antibody epitopes of Entamoeba histolytica galactose adhesin map to the cystein rich extracellular domain of the 170 kDa subunit. *Infect Immun* 1993; 61: 1772-1778), (Zhang T et al. Use of a recombinant 170 kDa surface Ag of *Entamoeba histolytica* for serodiagnosis of amebiasis and identification of immunodominant domain of the native molecule J. Clin. Microbiol 1992; 30: 2788-2792), (Schain DC, Salata RA, Ravdin JI. Development of amebicidal cell mediated immunity in gerbils *(Meriones unguiculatus)* immunized with the galactose inhibitable adherence lectin of *Entamoeba histolytica. J Parasitol* 1995; 81: 563- 568), Petri WA, Ravdin JI. Protection of gerbils from amebic liver abscess by immunization with the galactose-specific adherence lectin of *Entamoeba histolytica. Infect Immun* 1991; 59: 97-101), (Schain DC, Salata RA, Ravdin JI. Human T lymphocyte proliferation, lymphokine production, and amebicidal activity elicited by the galactose inhibitable adherence protein of Entamoeba histolytica. *Infect Immun* 1992; 60:2143- 2146), (Gaucher and Chadee 2000).

Another of the most studied membrane proteins, the serine-rich protein from *E. histolytica* (SREHP), after experimental animal immunization with this protein, with constituent peptides of the same as a DNA vaccine and expressed in attenuated live vectors such as S. *typhi* and *V. cholerae,* has proved a variable protection. (Zhang T, Stanley SL. Oral immunization with and attenuated vaccine strain of *Salmonella thyphimurium* expressing the serine rich *Entamoeba histolytica* protein induces an antiamebic immune response and protects gerbils from amebic liver abscess. *Infect Immun* 1996; 64 :1526.-1531), (Ryan ET, Butterton JR, Zhang T, Baker MA, Stanley SL and Calderwood, SB. Oral immunization with attenuated vaccine strain of *Vibrio cholerae* expressing a dodecapeptide repeat of the serine rich *Entamoeba histolytica* protein fused to the cholerae toxin B subunit induces systemic and mucosal antiamebic and anti-*V. cholerae* antibodies responses in mice. *Infect Immune* 1997; 65: 3118-3125), (Zhang T, Li E, Stanley SL. Oral immunization with the dodecapeptide repeat of the serine rich *Entamoeba histolytica* protein (SREHP) fused to the cholera toxin B subunit induces a mucosal and systemic anti-SREHP antibody response. *Infect Immun* 1995; 63: 1349-1355), (Zhang T, Cieslak PR, Foster L, Kunz Jenkins C, Stanley SL. Antibodies to the serine rich *Entamoeba histolytica* protein (SREHP) prevent amebic liver abscess in severe combined immunodeficient (SCID) mice. *Parasite Immunol* 1994c;16: 225-230). (Zhang T, Cieslak PR, Stanley SL. Protection of gerbils from amebic liver abscess by immunization with a recombinant *Entamoeba histolytica* antigen. *Infect Immun* 1994a; 62: 1166-1170), (Zhang T, Stanley SL. DNA vaccination with the serine rich *Entamoeba histolytica* protein (SREHP) prevents amebic liver abscess in rodent models of disease. *Vaccine.* 2000; 18: 868-874).

The 220 kDa lectin is a membrane protein, which is recognized, in more than 60% of the cases studied, by the sera of patients convalescing from amoebian liver abscesses (Meza, 1989). In order to know its immunogenicity, BALB/c mice were immunized by subcutaneous route with the native protein, a recombinant protein and peptides from this later obtained with treatments with urea-N chlorosuccinamidate. Immunized animals evidenced a state of protection against intrahepatic challenge (Martinez Gomez P, Perez Carrillo VL, Talamas Rohana P. Preliminary study of 220 kDa Lectin elicit immune response in hamster: possible vaccine candidate. Arch Med Res 1997; 28: S267-S268), (Talamas Rohana P, Schilie Guzman MA, Hernandez Ramirez VI, Rosales Encina JL. T-cell suppression and selective in vivo activation of TH2 subpopulation by the *Entamoeba histolytica* 220 kDa lectin. *Infect Immune* 1995; 63: 3953)

Another protein interesting as target for vaccine design is a 29/30 kDa cystein-rich protein. The coding sequence for this protein was determined in Torian BE, Flores BM, Stoeher VL, Hagen FS, Stamm WE. cDNA sequence analysis of a 29 kDa cystein-rich surface antigen of pathogenic E*ntamoeba histolytica.* Proc Natl Acad Sci USA 1990; 87: 6335-6362. Subsequently, this antigen was identified in the HM1-IMSS strain (Tachinaba H, Ihrara S, Kobayashi S, Kaneda Y, Takechi T, Watanabe T. Differences in genomic DNA sequences between pathogenic and non-pathogenic isolates of *Entamoeba histolytica* identified by polymerase chain reaction. *J Clin Microbiol* 1991; 29: 2234-2239) and its molecular characterization showed that it has an unusual cystein-rich domain comprising 18 aminoacids (Bruchhaus I, Tannich E. Analysis of the genomic sequence encoding the 29 kDa cystein rich protein of *Entamoeba histolytica. Trop Med Parasitol* 1993; 44: 116-118). It has been proved that such protein is immunogenic, since it was recognized by more than 80% of the sera of patients suffering from amoebian liver abscess (Flores B M, Reed SL, Ravdin JI, Torian B.E. Serological reactivities to purified recombinant and native 29 kDa peripheral membrane protein of pathogenic *Entamoeba histolytica. J Clin Microbiol* 1993; 31: 1403-1407). After immunizing animals with this protein, a 70 % protection was proved in an intrahepatic abscess model (Soong CJG, Torian BE, Abd Alla MD, Jackson TFHG, Gatharim V, Ravdin JI. Protection of gerbils from amebic liver abscess by immunization with recombinant *Entamoeba histolytica* 29 kDa antigen. *Infect Immun* 1995; 63: 472-477).

However, all prior experimental vaccines are based on immunization with a single constituent antigen of the parasite (adhesins), fact which limits their effectiveness, since the amoeba is a complex parasite with a great amount of adhesins and other proteins associated to its pathogenicity and virulence, therefore vaccinal preparations based on a single component will be insufficient to control the infection and its consequences, being necessary then to develop vaccines containing more than one virulence determinant in order to inhibit simultaneously several aggression mechanisms of the parasite. On the other hand, the use of vaccines based on a single component may favour the selection of mutants that would be subsequently resistant to immune mechanisms induced by the vaccination.

Recently the presence of a 112 kDa amoebian protein which is a surface molecule sharing adhesin and protease functions has been reported (Garcia Rivera G, Rodriguez MA, Ocadiz R, Martinez Lopez C, Arroyo R, Gonzalez Robles A, Orozco E. *Entamoeba histolytica:* a novel cystein protease and an adhesin form the 112 kDa surface protein. *Molecular Microbiology* 1999; 33: 556-568). The molecule corresponds to a heterodimer, constituted by a 78 kDa polypeptide with adhesin function and another 49 kDa polypeptide with cystein-protease activity, being each one codified by an independent gene. These genes were localized in the genome, in adjacent positions and separated only by a non-coding 188 bp sequence. (Garcia Rivera G, Rodriguez MA, Ocadiz R, Martinez Lopez C, Arroyo R, Gonzalez Robles A, Orozco *E. Entamoeba histolytica:* a novel cystein protease and an adhesin form the 112 kDa surface protein. *Molecular Microbiology* 1999; 33: 556-568). Up to this moment, this protein has not been used in the development of formulations with a specific use for the therapeutic, prophylaxis or diagnosis of diseases associated to *Entamoeba histolytica.*

### Disclosure of the invention

The object of this invention is the development of vaccinal compositions based on the use of a 112 KD surface protein from *Entamoeba histolytica* with prophylactic and therapeutic purposes regarding the infection caused by this protozoan, whether in formulations including the genes that integrate the same and administered as DNA vaccine or in formulations with the purified protein or fragments of the same.

Surprisingly, it has been observed that immunization with formulations containing the protein mixture or with formulations containing the gene mixture (DNA vaccines), produces a higher attenuation of the hepatic damage produced by intraportal challenge model in hamsters, as compared with that produced by each protein or each gene separately and this effect is significantly higher than that expected for the mixture, which indicates an enhancing or synergic effect in the protection against hepatic damage upon immunization with the mixture (Example 10).

This synergic effect of two amoebian proteins has not been reported before, so it is a new strategy to achieve new vaccinal formulations effective against amoebiasis.

The vaccinal preparations of the invention are constituted by the natural purified protein as well as by its 2 subunits (cystein protease and adhesin) obtained by recombinant means in *E. Coli,* used alone or in mixtures. These compositions use between 30 µg and 1 mg of protein, amounting this active principle to about 25% to 70% of the whole formulation. In the particular case of the mixture, the protein subunits were used in equal proportions, using doses including 10 and 1000 µg that amount to between 10-80 % of the whole formulation, depending on the formulation used.

Vaccinal formulations containing the genes coding the subunits with cystein protease and adhesin activities in an appropriate expression vector, using them alone in doses ranging between 5µg and 800 µg or preferably combined in 1:1 proportion in the same composition, with doses including between 10 µg and 800 µg of DNA and amounting to 25-80% of the whole formulation are also included within the scope of this invention.

It is also included within the scope of this invention the administration of the products of genes coding the subunits with cystein protease and adhesin activities, expressed in attenuated live vectors to be selected from the group comprising *Mycobacterium bovis,* BCG, *Salmonella sp, Vaccinia,* Adenovirus, Poliovirus wherein genes are introduced by means of genetic transformation using the appropriate plasmid vectors and which are administered at a dose level of attenuated live vector with excipients ranging between 20 µg and 100 µg, amounting this active principle to between 25% and 60% of the whole formulation.

The compositions included within the scope of this invention can be used for the prophylaxis and therapeutic treatment of human infection by *Entamoeba histolytica.*

With all compositions used, the induction of a specific humoral (Examples 2 through 6) and cellular (Examples 7 through 9) immune response in immunized hamsters was proved, as well as the inhibition of trophozoites adherence and cytopathic effect in cell lines under culture by the sera of immunized animals (Example 11).

Likewise, it is included within the scope of this invention the use of any of the vaccinal compositions by any route selected among those of the group comprising: ocular, otic, vaginal, rectal, endovenous, subcutaneous route and topical route on skin. Particularly, oral, intranasal, intradermal and intramuscular routes.

This invention addresses in a novel way the development of vaccines for the prevention and treatment of amoebiasis, starting from the use of formulations based on a protein or genes coding the same, achieving the stimulation of an effective immune response not only against the cytopathic effect caused by this microorganism (achieved by prior vaccinal candidates) but also against the intestinal colonization by such microorganism, a crucial element that had not been solved up to this moment, both for the prevention of clinical extraintestinal symptoms as well as for the effective therapeutic treatment of the disease.

This invention shall be described by means of the following specific examples.

### Examples of Embodiments of the Invention

### Example 1. Attainment of plasmids pcdna-Ehadh112 and pcdna-Ehcp112.

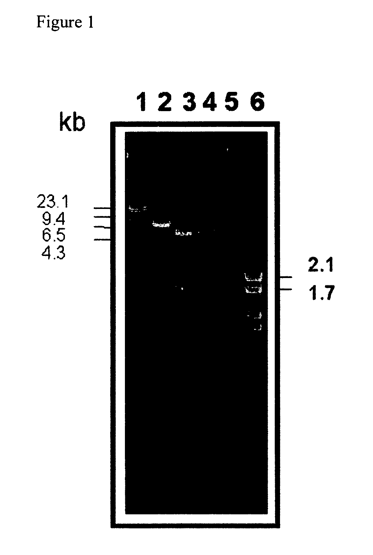

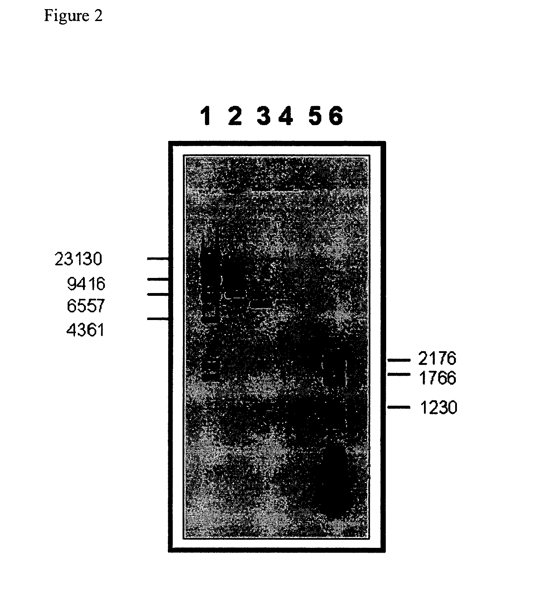

For the construction of both plasmids, the expression vector *pcDNA*_{*3*} (Invitrogen, San Diego, USA) was used; it was cut with restriction enzymes of the multiple cloning site, BamH I, EcoR I, for cloning cystein protease gene (1.3 Kb), while after digestion with Xba I, Apa I (Gibco, USA), it was used for cloning a fragment of the amoebian adhesin gene (1.7 Kb). The corresponding genic inserts were obtained by enzymatic restriction starting from the prior work carried out by Garcia-Ribera et al., wherein they obtained a 5.6 kb fragment that had two open reading frames forming the 112 kDa amoebian surface protein (Rivera-Rivera G, Avila A, Ayala P, Arrollo R, Rigothier MC and Orozco C. 1997. Identification and localization of the cell binding domain in the 112 kDa adhesin gene of *Entamoeba histolytica.* Arch. Med. Res 28s:164-165). The insert purification was carried out by adsorption into glass beads using a Geneclean II kit (BIO 101, USA) and then the inserts were bound to the corresponding vectors. The plasmids thus obtained were called *pcDNA-Ehcp112* and *pcDNA-Ehadh112* respectively. These constructions were introduced into a DH-5 strain of *Escherichia coli* and then they were purified using the columns of the Quiagen Giga-Plasmid Columns commercial kit (Quiagen, USA) following the instructions of the manufacturer's purification manual. The purity and concentration of the DNA thus obtained was determined by spectrophotometry in a DU 650 equipment (Beckman, USA), reading sample absorbance at 260/280 nm. The DNA was also visualized on 0.8% agarose gels pursuant to the method described by Sambrook et al (Sambrook, J., Fritsch, E.F. and Maniatis, Vol. 1989. Molecular Cloning: a Laboratory Manual. 2^{nd} Edition. Cold Spring Harbor Lab. Press. USA). The separated DNA was visualized by ultraviolet light irradiation at 310 nm and the results were registered in a GelDoc 1000 equipment (BioRad, USA). The insert was a fragment of 1.7 Kb out of a total of 2.0 Kb which form the adhesin gene; the fragment lacked the first 360 base pairs of the 5'-end of the gene, but included those as from the possible transmembrane regions up to the end of its 3'-end. In this case, the vector contributed the initial codon of translation. Said construction was used to transform competent E. *coli DH5a* bacteria. The recombinant plasmid was extracted from the bacteria by means of alkaline lysis purification and its identity was verified by digestion with the enzymes used for the cloning strategy. This digestion allowed the release of the adhesin gene having a molecular weight of 1.7 Kb from the 5.4 Kb vector (figure 1) and thus the selection of bacterial cultures having the construction of interest. In order to verify the identity of the *pcDNA-Ehadh112* construction, a sample was sequenced using an oligonucleotide that enabled reading the sequence as from the 5' cloning end and 200 bp more downstream (results not shown). The second vaccinal plasmid was constructed with the gene of the smaller subunit of the 112 kDa amoebian surface protein, the one corresponding to the protease. The resulting plasmid was called *pcDNA-Ehcp112.* Said construction was used to transform competent *E*. *coli DH5α* bacteria. The recombinant plasmid was extracted from the bacteria by means of alkaline lysis purification and its identity was verified by restriction analysis with the enzymes used in the cloning. This digestion enabled the release of the cystein protease gene having a molecular weight of 1.3 Kb from the 5.4 Kb vector (figure 2) in those cases where the construction of interest was found. In order to verify the identity of the plasmid *pcDNA-Ehcp112,* a DNA sample was analysed in an automatic sequencing equipment, in a similar way to the analysis carried out with the previous plasmid (result not shown).

### Example 2. L929 cell transfection and in vitro expression of polypeptides Ehadh112 and Ehcp112.

The plasmid constructions described in Example 1 were used.

Transfection assays were carried out pursuant to the protocols set forth in Gibco Transfection guide, BRL. Cells were grown on 35-mm plates (COSTAR, USA) until achieving a cell confluence of between 60 and 80 %. 2 g of the plasmid DNA to be transfected, diluted in 100 µl of medium (DMEM, USA) free from bovine foetal serum (Gibco, BRL, USA) and 25 µl of lipofectamine (Gibco, BRL, USA) diluted in 100 µl of medium free from serum were used per each plate. The plates were incubated for 6 hours at 37°C in a 5% CO₂ atmosphere. At 24 hours as from the commencement of the transfection, the medium of the plates was replaced by fresh medium with whole serum (DMEM, with 10% foetal serum, 100 µg/ml of ampicillin and 100 µg/ml of streptomycin) and 48 hours later 1µg/ml of DMEM medium of the selected antibiotic, geneticin (Gibco, BRL, USA) was added to the cell culture. For RT-PCR assays cell cultures grown in 100-mm plates until achieving 80-90 % confluence were used, and RNA was extracted from the same with trizol: guanidinium phenol-isocyanate. Finally it was dissolved in 30 µl of H₂O-DEPC and incubated with DNase free from RNase, (RQI Promega) in a thermal bath at 37 °C during 30 min. To determine sample concentration, absorbance was read at 260 nm. RNA was stored precipitated with 70% ethanol at -70 °C, in 5 µl aliquots. For cDNA synthesis, PCR tubes were used, wherein 5 µl of whole RNA and 100 ng of the antisense oligo in a final volume of 12 µl were added and then the tubes were incubated at 70° C for 10 min. At the end of such period they were placed again in the ice bath and 4 µl of First Strand Buffer Kit 5X (Kit), 2 µl of 0.1 M DTT (Kit), 1µl of 10 mM dNTP Mix (Kit) were added. The sample was homogenized and incubated at 42° C for 2 min. Then 1 µl (5 units) of SuperScript II enzyme (Gibco- BRL Kit) was added and it was incubated for 50 min at 42° C. The reaction was obtained placing the tubes at 70° C during 15 min.

The reaction product was treated with RNase H. Finally the samples were stored at -20° C.

The antisense oligo sequences used were the following:
*Ehadh112:* 5'- TGCTTGAGTATAAGCAG 3'
*neo*^{*r*}: 5' TTAGGAGAACTCGTC -3'

Per each DNA fragment which amplification was intended, 10 µl of Taq Polymerase Buffer 10X, 8 µl of MgCl2, 200 ng of the sense oligo and 200 ng of the antisense oligo, 2 µl of the product of RT-PCR, 2 µl of dNTP and 0.6 µl (5 U/µl) of Taq polymerase enzyme were added to a final volume of 100 µl. The initial temperature of DNA denaturalisation used was 94° C for 5 min, then there followed 30 denaturalisation cycles, alignment of oligos and extension (94° C for 30 sec., Tm for 35 sec. and 72° C for 1 min, respectively). Then, 7 min of final extension at 72° C and a maintenance temperature of 4° C were programmed.

The sense oligo sequences used were the following:
*Ehadh112*: 5'- ACTTGATTTTGAAAAGG -3'
*neo*^{*r*}: 5' ATGATTGAACAAGATGG -3'

To carry out indirect immunofluorescence assays, transfected L-929 cell cultures were grown on zero cover slips for 24 hours and the cells were fixed with a cold acetone-methanol solution (v/v) that was let standing for 2 min. Fixed cells were incubated for 1 hour at 37° C with serum from hamsters immunized with the plasmid constructions under study, at a 1:20 dilution. Afterwards, the cover slips were incubated with the second goat anti-hamster IgG antibody labelled with fluorescein, at a 1:200 dilution. Finally they were examined through a Nikon Diaphot 200 microscope coupled to a MRC 1024 confocal laser scan (BioRad, USA). Once the vaccinal plasmids were obtained and before starting the direct inoculation in experimental animals, with the purpose of verifying in vitro that these constructions were capable of replication in an eukaryotic cell, of transcription and finally of expressing the gene product cloned in each, mouse fibroblast were selected as eukaryotic model system. Clones surviving after 2 weeks of sustained treatment were selected, at the moment when non-transfected cultures died under the same selective agent concentration. The use of geneticin enabled selection of those cells that were able to incorporate the plasmids of interest. Non-transfected cells died since they lacked such gene.

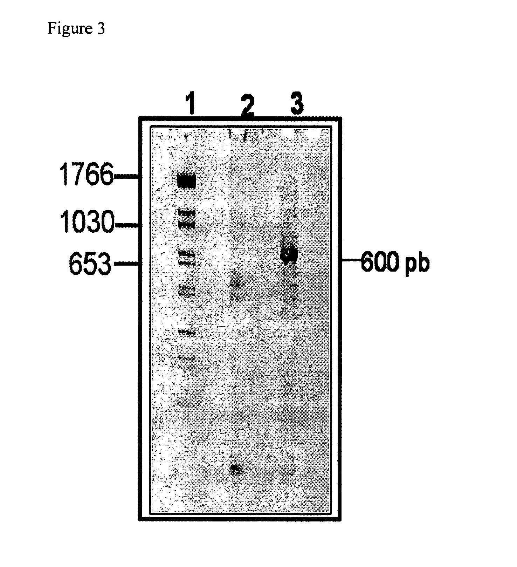

Starting from the RNA of transfected cells, it was proved that both cultures have been successfully transfected and adhesin gene transfection was being produced within them. The expected transcript according to the oligonucleotides used had about 600 bp. In RT-PCR product analysis by electrophoresis (figure 3), this band was selectively observed for the RNA of the culture transfected with *pcDNA-Ehadh112.*

L929 fibroblast cultures, independently transfected with plasmids *pcDNA-Ehcp112, pcDNA-Ehadh112 and pcDNA*_{*3*}*,* were grown on cover slips, to verify by indirect immunofluorescence assays the presence of amoebian proteins and to determine their location.

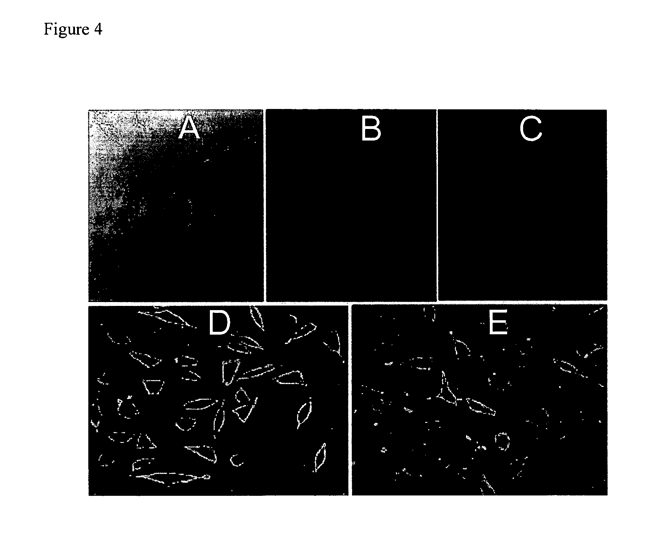

As a result of this assay, a fluorescent staining was observed on the surface of the cells transfected with the plasmid that had the amoebian gene inserts (*pcDNA-Ehcp112, pcDNA-Ehadh112),* and no fluorescent staining was observed in the same cells used as control to which the first antibody had not been added, nor in those cells transfected with the *pcDNA*_{*3*} vector (figure 4).

Plasmid capacity to transcribe and express their genes in eukaryotic cells from L929 murine fibroblasts was evaluated as a first step, before studying the immune response that their gene products were capable of inducing.

Fibroblasts were successfully transfected with both plasmids independently. The presence of the transcript for adhesin gene was evidenced and the existence of a membrane protein on the surface of the fibroblasts transfected with both plasmids was observed by immunofluorescence.

Both genes were cloned including the sequences of membrane anchoring and even though no sequence containing the characteristics of the leading peptide was found for the adhesin gene, at least in the case of the protease, it was included.

### Example 3. Study of humoral immune response induced in hamsters with plasmid pcDNA-Ehadh112.

The plasmid obtained and purified as shown in Example 1 was used. For this experiment, male hamsters, 4-5 weeks of age, allocated into 10 groups of 8 animals each were used. Immunizations were performed every 20 days by intradermal route at the base of the tail. In all the cases the inoculum had a volume of 100 µl and was applied three times with 1-ml syringes. 4 groups were inoculated with the plasmid *pcDNA*_{*3*}*-Ehadh112,* at the following doses 5 µg, 50 µg, 75 µg and 100 µg, with the plasmid *pcDNA*_{*3*}*-Ehcp112.* 2 groups were selected as controls, one was inoculated with the vector *pcDNA*_{*3*} used in the construction of the previous plasmids, and the other with sterile PBS, a solution used as vehicle for the inocula. Blood extraction was performed by punction at the retro-orbital sinus, and it was kept at -20° C until used. Sera were tested by Western blotting against whole protein extracts from amoebas, which were obtained pursuant to the method described by Avila et al. (Avila, E.E., Garza-Garza, M. and Calderon, J. 1985. *Entamoeba histolytica* and *E. invadens* sulphydryl-dependent proteolytic activity. J protozool. 32:163-166) and by Calderon and Avila (Calderon, J. Y Avila, E. 1986. Antibody induced caps in Entamoeba histolytica: isolation and electrophoretic analysis. J. Infect. Dis. 153:184-193). The electrophoretic separation was performed pursuant to the method described by Laemmli (Laemmli, U.K. 1970. Cleavage of structural proteins during the assembly of the head of bacteriophage T-4. Nature 277:680-685) and modified by Avila (Avila, E.E 1982. Identificación de las proteínas de membrana plasmática y del "cap"inducido por anticuerpos en Entamoeba histolytica. Magister Thesis in Sciences (Cell Biology). Centro de investigación y Estudios Avanzados del IPN. Mexico, D.F). 12% gels were used. The gels were run at 100 V.

For the transference of proteins separated by electrophoresis, the technique described by Towbin et al was used. (Towbin, H., Staehelin, T. Y Gordon, G. 1979. Electrophoretic transfer of proteins from polyacrilamide gels to nitrocellulose sheets: procedure and some applications. *Proc. Nat. Acad. Sci. USA.* 76: 4350-4354). The nitrocellulose membrane was blocked by 2% PBS-BSA, and it was incubated with the first antibody (mouse monoclonal antibody IgM directed against the 112 kDa protein or hamster immune serum) overnight at 4° C with gentle agitation. The second specific antibodies (mouse anti-IgM with peroxidase and hamster anti-IgG with peroxidase, Zymed, USA), were left at room temperature, with agitation for 2 hours. Non-fixed antibody was eliminated by two consecutive washes with a 20 mM phosphate buffer solution (10 mM Na₂HPO₄, 10 mM NaH₂PO₄, pH 7.4 and 150 mM NaCl) and once with a 50 mM phosphate buffer solution (25 mM Na₂HPO₄, and 25 mM NaH₂PO_{4,} pH 7.4). Protein recognition by anti-sera was developed with 4-chloro-1-naftol (Sigma, USA). ELISA assays were performed by a method similar to that described by Zhang and Stanley, (Zhang T, Stanley SL. DNA vaccination with the serine rich *Entamoeba histolytica* protein (SREHP) prevents amebic liver abscess in rodent models of disease. *Vaccine.* 2000; 18: 868-874). 96-well plates (COSTAR; USA) were coated with 50 µl (1x10⁴ amoebas) of a solution of trophozoites inactivated with heat in PBS overnight at 4° C. The following day they were washed 4 times with 150 µl/well of PBS-tween at 0.05 % and then 100 µl of a blocking solution (2% PBS-BSA) were added to each well; the plates were incubated for 1 hour at 37° C; then, the first antibody, 50 µl of hamster sera at a 1:500 dilution in PBS-BSA at 1% was added and it was left overnight at 4° C. 50 µl of the second antibody, hamster anti IgG with peroxidase at 1:2 000 dilution in 1% PBS-BSA were added. The plates were incubated 1 hour at room temperature and then they were carefully washed and developed with orthophenylendiamine. The reaction was stopped with 25 µl of 2.5 M H₂SO₄. The results of the colorimetric reaction were read in an ELISA reader at an optic density of 492 nm. All samples were applied by duplicate.

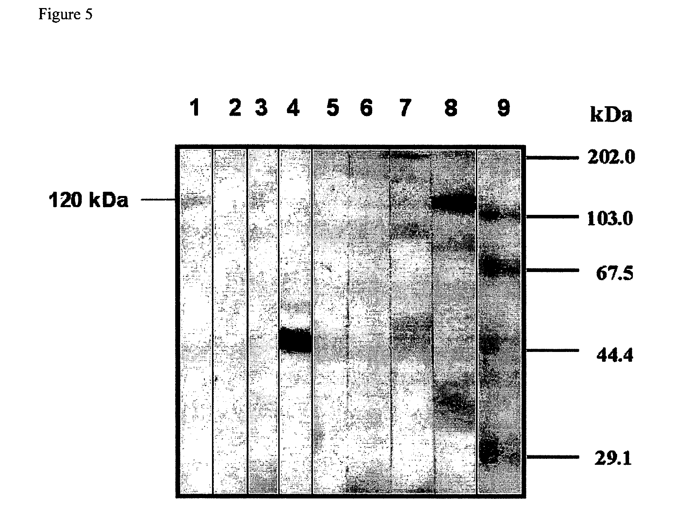

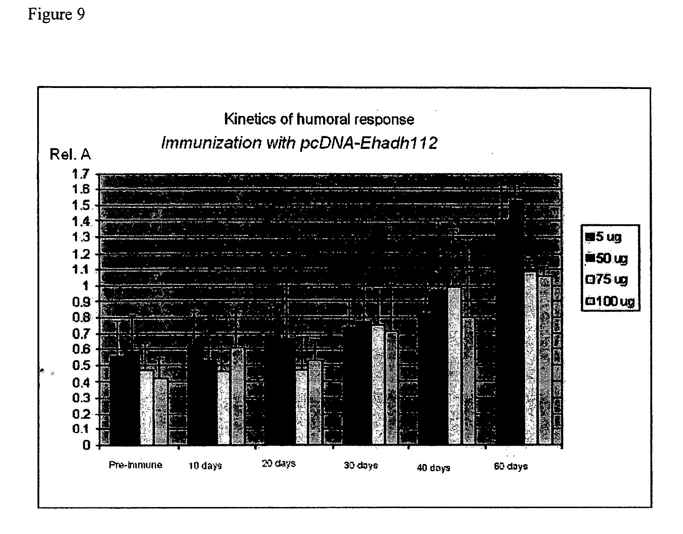

The sera of animals immunized with pcDNA-Ehadh112 recognized a band with a molecular size of 120 kDa, which coincided with the length of the band recognized by an anti-112 monoclonal antibody. Other signs of unspecific recognition were observed. The pool of sera from the control group did not produce a signal at that point, and neither did the preimmune sera (figure 5). On the other hand, the serum of hamsters inoculated with the DNA of interest recognized the 31 kDa recombinant adhesin that preserves the adherence domain and the capacity of being recognized by the monoclonal antibody. Pre-immune sera did not produce a signal against the recombinant protein. Serum from hamsters immunized with the different doses of the plasmid pcDNA-Ehadh112 was sampled periodically, and said serum was used to determine, by ELISA, the presence of IgG antibodies against membrane proteins of E. histolytica (figure 9).

The result of this experiment proved that the level of anti-amoebian seric antibodies implied an increasing trend; however, no significant difference was found between the levels registered at zero time and those detected at subsequent extractions. Only the serum of the last extraction, sampled before slaughter and one week after challenge, showed differences with the levels of their respective preimmune sera.

### Example 4. Immunofluorescence on amoebian trophozoites.

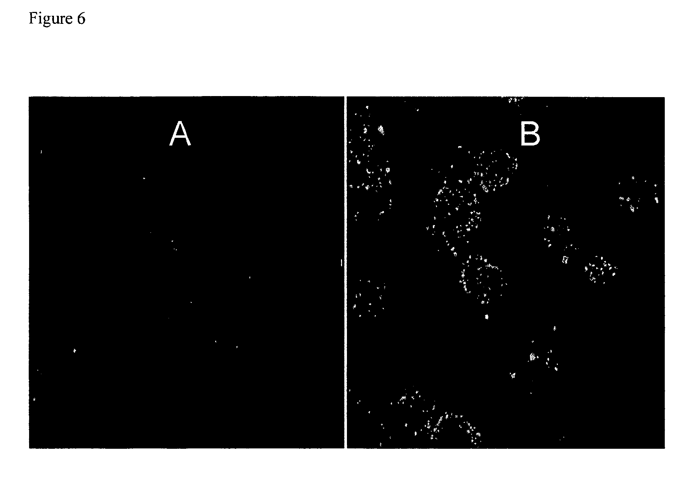

Immune sera obtained as described in example 3 were used. Trophozoites from HM1-IMSS strain were cultured on covers slips for 24 hours and washed with PBS to remove the culture medium; they were fixed with 2.5% paraformaldehyde (Sigma, USA) dissolved in PBS; then they were permeabilized with 0.5% triton X-100 (Sigma, USA) for 10 minutes at room temperature. The indirect immunofluorescence assay was carried out on fixed cells as described previously in example 2. In order to determine the recognition of a surface protein on the amoeba by the immune serum, amoebian trophozoites of the strain *HMI-IMSS* were cultured with immune serum of hamster inoculated with plasmid *pcDNA-Ehadh112* and control serum (from animals inoculated with the vector). The sheets were observed by confocal microscopy. As a result of this indirect immunofluorescence assay, it was observed that the trophozoites incubated with control sera did not show fluorescence and those incubated with immune serum, after the third extraction, showed fluorescence, specially on the surface of the amoebas (figure 6).

### Example 5. Study of humoral immune response induced in hamsters with plasmid pcDNA-Ehcp112.

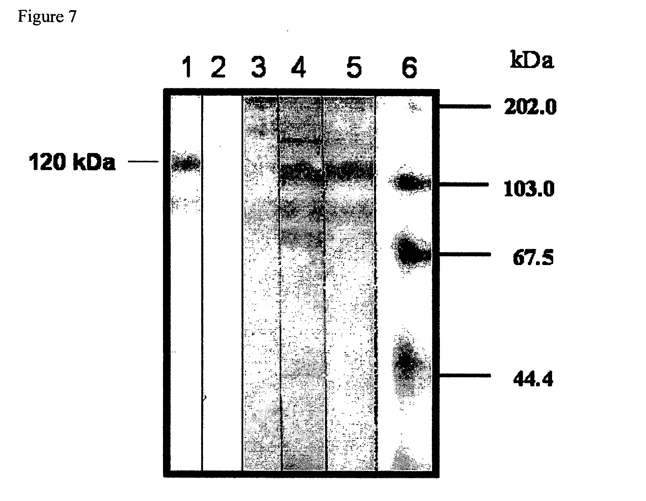

The plasmid obtained and purified as shown in Example 1 was used. The same immunization and blood sampling protocol used in Example 3 was used in this case. 3 groups were inoculated with the plasmid *pcDNA*_{*3*}*-Ehcp112,* at doses of 50 µg, 100 µg and 200 µg. The procedures used for Western blot and ELISA assays were those described in Example 3. Hamsters were immunized with different doses to study the induction of an immune response by in *vivo* expression of the 112 kDa amoebian protease. When the serum of hamsters immunized with *pcDNA-Ehcp112* was tested by Western blotting, a recognition signal was obtained. It agreed with that detected in the lane corresponding to the positive control and none was detected when preimmune serum was used as the first antibody (figure 7).

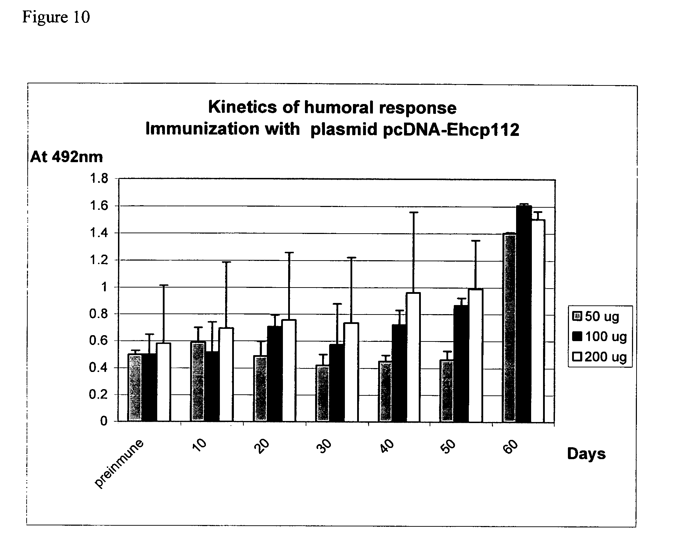

With the serum extracted every 10 days as from the commencement of the immunization scheme, a study of the trend of IgG antibodies level along the experiment was performed (figure 10). As a result of this experiment, a response was observed wherein after a first increase in antibodies titter, it falls to be subsequently increased up to somewhat higher levels. Significant differences were registered between the values of preimmune serum and those obtained at the end of the experiment. This did not happen with the sera of prior extractions. No statistically significant differences were found in the levels reached between the assayed doses.

### Example 6. Study of humoral immune response induced in hamsters with plasmids pcDNA-Ehadh112 and pcDNA-Ehcp112.

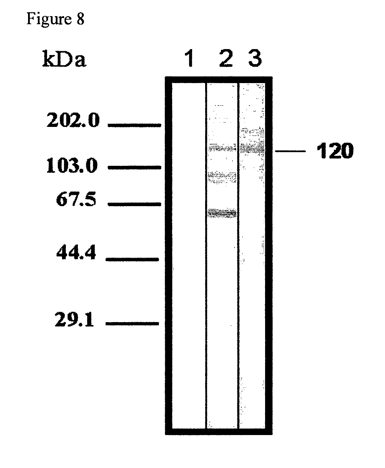

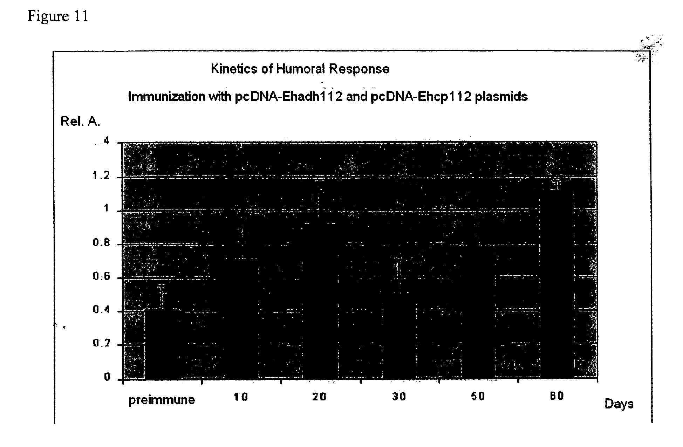

The plasmid used was that obtained and purified as shown in Example 1. The immunization and blood sampling protocol used was the same described in Example 3. In this study a plasmid mixture comprising 50 µg of each plasmid was used. The procedures used for Western blot and ELISA assays were those described in Example 3. The immune serum extracted from these animals was used to detect the presence of specific IgG antibodies against the amoebian protein by Western blot (Figure 8). The pool of sera from animals immunized with both plasmids gave a positive recognition signal at the observed height in the positive control; this signal was not observed in pre-immune sera. The serum extracted to these animals every 10 days enabled us to carry out a study of the kinetics of humoral response. The determination of the levels of IgG seric antibodies capable of recognizing proteins of whole trophozoites was carried out by ELISA, following the same procedure used in prior kinetic studies (figure 11). The results enabled us to observe a biphasic behaviour in the humoral response of this group of animals. After an increase in antibody titter during the first two extractions, a decrease detected at day 30 post commencement of the scheme was detected, i. e., after the second dose, to be increased again after the third dose and up to the end of the study. Data analysis revealed a statistically significant difference between the level of antibodies found in pre-immune serum and those levels reached in each subsequent extraction, as from the extraction carried out on day 10 post-first doses up to that performed the second month after the commencement of the study. It was found that the extraction carried out on day 30 of the scheme was an exception in this regard, since no significant difference with preimmune serum was reported. The results confirmed those found in the Western blot about the immunogenicity of amoebian proteins. It was observed that the titter of IgG antibodies was increased as the animals were receiving immunizations. Likewise, the efficacy of the route selected and the dose range assayed to study the induction of an immune response was evidenced. These results suggested, in the first place, that plasmids *pcDNA-Ehadh112* and *pcDNA-Ehcp112* injected by transdermal route in hamsters, transfected host cells, probably into keratinocytes and may be in cells of the cutaneous immune system. In the second place, it was suggested that these cells achieved the endogenous expression, both of the amoebian adhesin and protease. In the third place, it was indicated that said constructions, at the dose and by the administration route selected, turned to be immunogenic in hamsters. And in the last place, it was observed that the mixture of both plasmids for its simultaneous administration does not depress the response observed for each one independently.

### Example 7. Study of cellular immune response induced by plasmid pcDNA-Ehadh112.

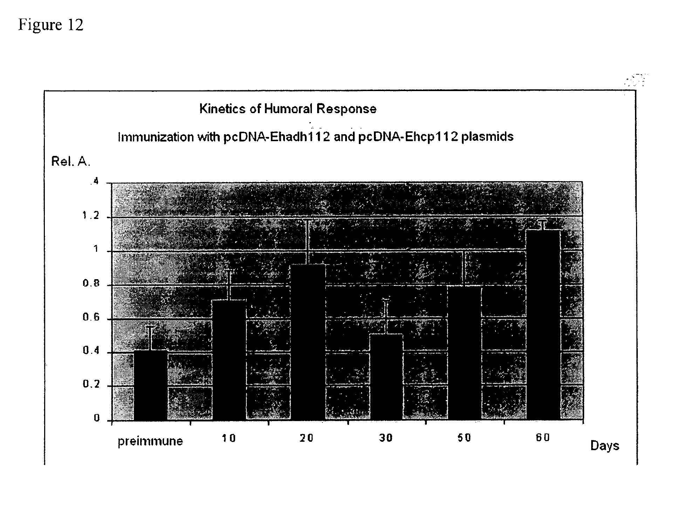

In order to study the immune response mediated by cells, a cell lymphoproliferation assay was carried out, using the plasmids described in Example 1 and the immunization scheme described in Example 2. The lymphoproliferation assays were carried out as described by Lotter et al. (Lotter H, Zhang T, Seydel KB, Stanley SL Jr; Tannich E. Identification of an epitope on the *Entamoeba histolytica* 170 kDa lectin conferring antibody-mediated protection against invasive amebiasis. *J Exp Med* 1997; 185: 1793-1803). The same were carried out with whole cells from the spleen of hamsters under study. Lymphocytes extracted from the spleen of the animals under study and DMEM medium supplemented with foetal serum were used in these assays. Lymphocytes were adjusted to 2x 10⁶ cell/ml with full RPMI. Concanavaline A (Pharmacia, Sweden) at 0.25 mg/ml (unspecific mitogen) and amoebas belonging to *HMI-IMSS* strain, inactivated by heat and adjusted with whole DMEM to 4 x 10⁵ cell/ ml, were used as antigens. In flat-bottom, 96-wells culture plates (COSTAR; USA), 100 µl/well of the lymphocyte suspension from each hamster were placed, that were put in contact with 3 different concentrations of specific antigens (4x10⁵, 2x10⁵, 1x10⁵ trophozoites/well) and with 0.25 mg/ml of Concanavaline A. All studies were carried out in duplicate. The plates were incubated for 72 hours at 37° C in a 5% CO₂ atmosphere and after 56 hours as from the commencement of the assay, 20 µl/well (1 µCi) of a tritiated timidine solution in DMEM (0.5 mCi /10 ml DMEM) were added. Once this period had elapsed, the plates were read. In order to study the behaviour of the cell component of the response in the animals immunized with plasmid *pcDNA-Ehadh112,* cell lymphoproliferation studies were performed (Figure 12) The results show that the intradermal injection in hamsters of the construction carrying the amoebian adhesin gene was immunogenic, thus creating an immune response with the participation of the cell component. The lowest amount of inactivated trophozoites used for in vitro restimulation was 10 000 amoebas per well. Data analysis was performed with CPM values reached by control cells as compared against CPM values of the cells of the immune group, per each group. It was evidenced that the animal groups receiving the lowest doses of the plasmid (5 and 50 µg), showed a smaller response than those receiving the highest doses, in a direct relationship. The group immunized with the 75 µg dose showed a stimulation index over 8 and the group immunized with the maximum dose, over 11, while the indexes of the groups receiving the lower doses were under 3. The control group receiving high doses of the vector was equally challenged, and showed no lymphocyte proliferation.

### Example 8. Study of cellular immune response induced by plasmid pcDNA--Ehcp112.

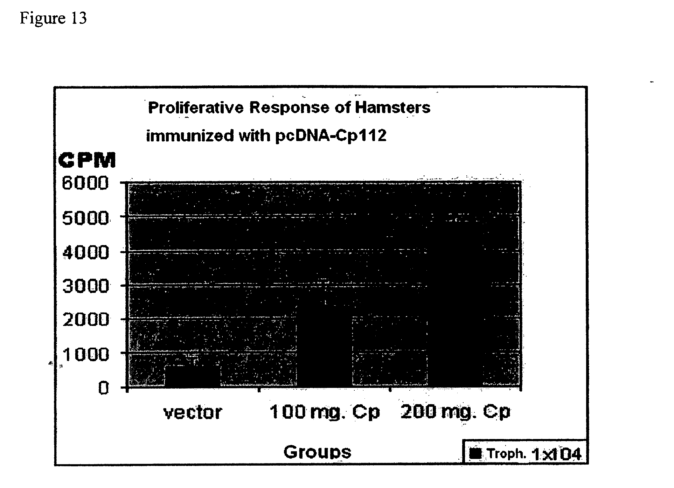

A cell lymphoproliferation assay was carried out, using the plasmids described in Example 1 and the immunization scheme described in Example 2. The procedure followed was that described in Example 7. In order to study the cell component of the immune response in the immunized animals, a spleen ablation was performed at the end of the experiment, and the splenocytes were used in a lymphoproliferation assay. The animals immunized with 100 µg of the vector were used as control group; this group was challenged in the same way as the groups under study (figure 13). Data analysis was performed with the values obtained from the comparison of CPM values reached by control cells against CPM values of the cells of the immune group, per each group. The animals immunized with 200 µg had a response that duplicated that found in hamsters immunized with 100 µg. However, lymphocytes from both groups showed a cell proliferation to *in vitro* stimulation that was not found in the control group. On the other hand, the highest indexes were obtained with a restimulation of 10000 trophozoites per well, 4.1 for the 100µg dose and 8.3 for the 200µg dose. It was found that the value of this ratio for those receiving 100 µg, decreased to 3.4 when the assay was performed with 20000 trophozoites and to 2.2 when the amoebas concentration was duplicated. For those inoculated with 200µg of *pcDNA-Ehcp112,* the values of this ratio decreased as the concentration of trophozoites was increased, and it was 5.1, for stimulations with 20000 and 4.3 for stimulations made with 40000 amoebas.

### Example 9. Study of cellular immune response induced by plasmids pcDNA-Ehadh112 and pcDNA-Ehcp112.

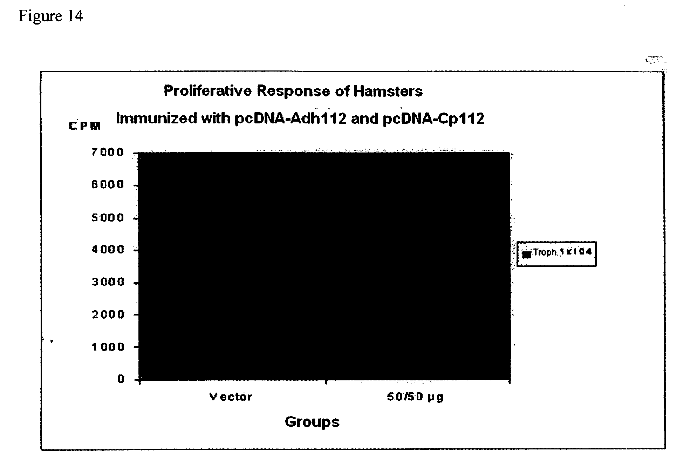

A cell lymphoproliferation assay was carried out, using the plasmids described in Example 1 and the immunization scheme described in Example 2. The procedure followed was that described in Example 7. The analysis of the cellular response was also performed in this group following the same protocol as that used in the study of each plasmid independently (figure 14). The result of this experiment evidenced a T-cell proliferation in the immunized group against the control group, with a proliferation ratio of control group to immune group of 9:1. It was also observed that the concentration of 10000 trophozoites per well was the ideal concentration to evidence this result. The immunogenicity study of these amoebian proteins expressed in hamster was extended to the cellular immune response by means of lymphoproliferation assays. A comparative analysis between the groups that received independently a single plasmid at the different doses and those receiving the mixture of plasmids was carried out. The results obtained in these experiments enabled us to conclude that the intensity of the responses found had a direct relationship with the assayed doses in each group. This happened with the two plasmids under study, finding the highest CPM values in the cells of those animals receiving 100µg of the plasmid *pcDNA-Ehadh112* and in the cells of those receiving 200 µg of pcDNA*-Ehcp112.* However, the proliferative response was higher in the group immunized with the fragment of the adhesin gene than with the protease gene. The first group had counts over 11000, for an immunization dose of 100 µg, while the second, for a dose of 200 µg, had values under 5 000 CPM. If we analyse the values corresponding to the group immunized with a mixture of 50 µg of each of the plasmids under study, we find that CPM values (6829) were higher than those obtained for the 50 ug dose of the group immunized with *pcDNA-Ehadh112* (1599 CPM), and even higher than those of the group that received 200 ug of *pcDNA-Ehcp112* (4812 CPM). This analysis suggests the existence of a synergism in cellular response, since the combination of both plasmids induced a lymphoproliferative response higher than the independent administration of each of them, even at higher doses.

### Example 10. Determination of the protection induced by plasmids pcDNA-Ehadh112 and pcDNA-Ehcp112, against the intraportal challenge in the animals under study.

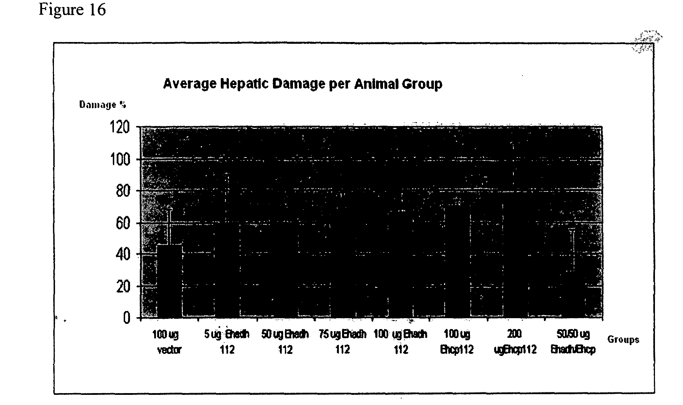

The plasmids described in Example 1 and the immunization scheme described in Example 2 were used in this study. Once the immunization scheme described in Example 2 was completed, the hamsters were challenged with virulent amoebas of the HM1:IMSS strain Under the effect of anestesal, the animals were practised a medial longitudinal incision than let the intestinal ansa be displaced in order to enable visualization of the hepatic ilium. Then 100 µl (1.5x10⁵ trophozoites) of a dilution of virulent amoebas were injected in the portal vein. The haemorrhage was stopped with gelfoam. Ansae were placed again in the intestinal cavity and the abdominal wall was sutured with 3.0 silk. A week after challenge the animals were slaughtered and their livers were removed in order to photograph the same and evaluate the percentage of hepatic damage. In order to evaluate the protection degree granted by the immunization performed, the animals were challenged with virulent trophozoites directly injected in the portal vein; they were slaughter 7 days after challenge and the percentage of hepatic tissue damaged as regards the total weight of the organ was calculated. As it can be observed in figure 16, immunizations performed with a single plasmid injected independently and at the different dose levels were not able to reduce the hepatic damage caused by the amoebian infection; no significant differences were found between these groups. However the group immunized with the mixture of these two plasmid constructions achieved a decrease statistically significant as regards the control group. Damage reduction amounted to 50% and it was notable, both regarding the decrease of the area with abscesses as well as the decrease in the total weight of the organ. The immunization with naked DNA using the two genes that code for the 112 kDa amoebian adhesin, when combined and separately inoculated, was capable of generating an immune response, both humoral and cellular; however, the protective capacity of such response had not been evaluated yet. For that purpose, *in vivo* virulence studies were carried out in young hamsters. It was evidenced that protection was not achieved by any of the doses studied for the plasmids (*pcDNA-Ehadh112* and *pcDNA-Ehcp112*) when administered independently. However, the group that was administered the mixture of both plasmids achieved a partial protection of 50%. The animals showed a decrease in the number and size of the abscesses. These low levels of protection found, as regards similar studies, might be due in part to the model of hepatic amoebiasis model. 150000 virulent trophozoites, after a recent passage through hamster liver, were directly inoculated in the portal vein.

### Example 11. In vitro virulence study.

With the immune sera obtained as described in Example 2, studies on inhibition of adherence to human erythrocytes and to cells obtained from dog kidney were carried out. Human erythrocytes were obtained by venous punction and Alvester solution was used as anticoagulant. (Garvey, J.S., Cremer, N.E. and Sussdorf, D.H. (1977) Reagents. Chap IV In: Methods in Immunology. Benjamin (Ed) Inc. Reading. Massachusetts 3rd edition). The blood sample was centrifuged at 1500 x g for 10 minutes, and then the erythrocytes were washed three times with the same solution and were resuspended in TYI-S-33 medium without serum. Erythrocytes were used fresh or up to 5 days after their extraction. (Garcia Rivera G, Rodriguez MA, Ocadiz R, Martinez-Lopez C, Arroyo R, Gonzalez Robles A, Orozco E. *Entamoeba histolytica:* a novel cystein protease and an adhesin form the 112 kDa surface protein. *Molecular Microbiology* 1999; 33: 556-568). Each assay was performed in triplicate. The 100% adherence was assigned to those cases where the amoebas were not in contact with hamster serum. In a similar way to the procedure used in the prior example, virulent trophozoites of the HM1-IMSS strain were harvested in their log phase of growth and were adjusted in TYI-S-33 culture medium without serum to a concentration of 1x106 trophozoites/ml; 600 µl were incubated with anti-hamster serum at a 1:250 dilution for 20 minutes at 4° C, in 15-ml Falcon tubes. Subsequently, the content of each tube was added over monolayers of MDCK cells, cultured in 24-well chamber dishes. The chambers were placed in an incubator at 37° C for 3 hours, until the cell monolayer of the control well was completely phagocyted by the amoebas. Immediately the chambers were placed at 4° C for 15 min and then, by means of washes with cold PBS, all the amoebas were removed. The rests of the monolayer were stained with 1% methylene blue in borate buffer at 37° for 30 minutes. Colour was measured in a spectrophotometer at 660 nm (Garcia Rivera G, Rodriguez MA, Ocadiz R, Martinez-Lopez C, Arroyo R, Gonzalez Robles A, Orozco E.

*Entamoeba histolytica:* a novel cystein protease and an adhesin form the 112 kDa surface protein. Molecular Microbiology 1999; 33: 556-568).

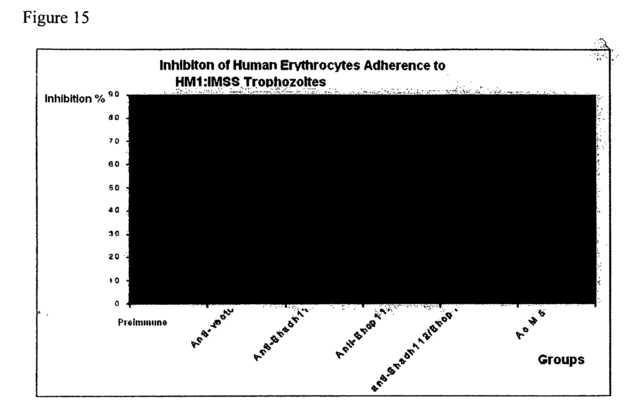

In order to continue characterizing the immune response, *in vitro* virulence studies were carried out to determine if those antibodies were capable of blocking the adherence of virulent amoebas of HM1-IMSS strain to human erythrocytes. The study was a comparative study between animals immunized with the plasmids containing adhesin genes, protease genes and the mixture of both genes and the group immunized with the vector. A pooling was made with the sera of those animals that better responded to each plasmid inoculation. This was performed according to the results of ELISA assays carried out with the sera of each of the hamsters (figure 15). This experiment evidenced an inhibitory effect of immune sera upon the adherence of virulent amoebas to human erythrocytes, comparable to the values of the positive control. The monoclonal antibody achieved a 75.6% inhibition; the serum of hamsters immunized with plasmid pcDNA-Ehadh112 achieved a 66.9%; the serum of the group injected with pcDNA-Ehcp112 a 59.8% and finally, the serum of hamsters immunized with both plasmids achieved a 65.2%. The serum of animals inoculated with the vector reached only 6.8% inhibition. The statistical analysis of data showed that there is a significant difference between the adherence inhibition achieved with pre-immune serum and with the positive control of the experiment, the Mab 5 antibody. Similarly, a difference was observed between the values achieved by pre-immune sera (A) and immune sera (C, D, E), as well as between the sera of animals immunized with the vector (B) and with the sera C, D and E. On the other hand, the study revealed that there are no significant differences between the different inhibition degrees caused by immune sera (see figure 16).

In order to study the capacity to inhibit amoebian adherence that the immune sera of the three animal groups inoculated with the different antigens may have, the assay was performed with human erythrocytes. It was found that the three groups of immune sera, the groups receiving the plasmids pcDNA-Ehadh112, pcDNA-Ehcp112 and the mixture of both, showed high inhibition levels, comparable to those showed by mAb112; in spite of the low antibody levels achieved after DNA immunization. These results indicate that even though the antibody response was low, the degree of specificity reached was not, since antibodies in low titters generated against only one amoebian adhesin achieved a high percentage of decrease in the adherence to human erythrocytes.

The capacity of those seric antibodies to inhibit, at least partially, the cytopathic effect of the amoebas on MDCK cells was also studied. The study consisted on evaluating the destruction produced on each monolayer and, in that way, calculate the inhibition of this process in the wells wherein serum had been added as compared with the wells without this addition. These laters were assigned 100 % cell destruction (data not shown). The result of this assay showed a value of 23.5% inhibition for the monoclonal antibody, 2.2% for pre-immune serum and 15.7% for the serum of hamsters immunized with the vector, that as previously noted, are our controls. The cases corresponding to sera immunized with plasmids pcDNA-Ehadh112 and pcDNA-Ehcp112 showed values of 29.2% and 24.7 % respectively; the sera of hamsters immunized with the mixture of plasmids showed a value of 33.7%. The statistical analysis of these data showed significant differences between the inhibition achieved by pre-immune and immune sera, but that was not the case between immune sera of animals injected with plasmids pcDNA-Ehadh112, pcDNA-Ehcp112 and the mixture in equal proportion of both plasmids. These results let us conclude that the immune sera of hamsters immunized with the plasmid constructions were capable of inhibiting, at least partially the destruction caused by amoebas on monolayers of MDCK cells.

### Example 12. Immunization with a fragment of the 112 kDa amoebian adhesin.

Based on prior works carried out by Garcia-Ribera et al. wherein they obtained a 5.6 kb fragment that had two open reading frames forming the 112 kDa amoebian surface protein (Rivera-Rivera G, Avila A, Ayala P, Arrollo R, Rigothier MC and Orozco C. 1997. Identification and localization of the cell binding domain in the 112 kDa adhesin gene of *Entamoeba histolytica.* Arch. Med. Res 28s:164-165), a fragment of the gene of Ehadh112 protein coding for the polypeptide that contains the last 240 aminoacids of the carboxyl-terminal end was obtained by enzymatic restriction. This fragment was cloned in the commercial expression vector pRSET (Invitrogen, USA) between Pvu II and BamH I restriction sites. The plasmid obtained was called pEhadh240.

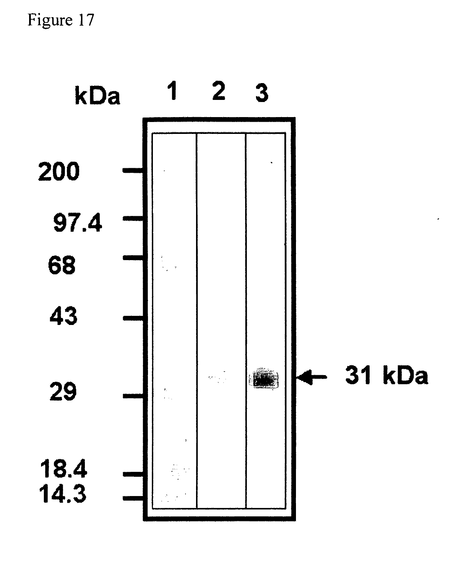

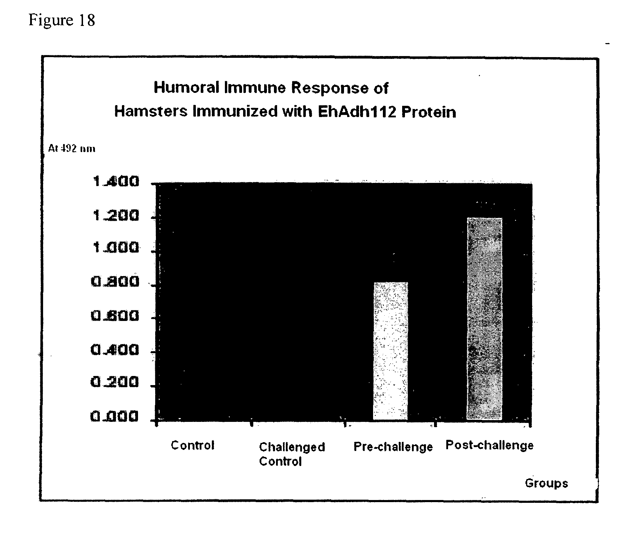

The plasmid obtained was introduced in E. Coli, strain BL 21 (DE3) and taking as starting point the cultures induced with isopropyl β-D-galactopiranoside (IPTG), the purification of the protein was carried out under denaturalising conditions by means of an affinity column Ni+2- NTA (Quiagen, USA), following the instructions of the manufacturer's manual. An experiment wherein 6 male hamsters, 4-5 weeks of age, were immunized with 120 µg of the recombinant protein Adh112 by subcutaneous route was carried out. The animals were administered 3 doses at a 21-day interval, and 6 hamsters inoculated with PBS were used as control. One week after the last immunization, all the animals were challenged with virulent amoebas. During the whole development of the study the animals were administered sterile food and water. Blood extraction was performed by punction at the retro-orbital sinus, and samples were kept at -20° C until the moment of use. The challenge was like the one described in Example 10. Western blot and ELISA assays were performed as described in Example 2. As a result of this experiment, the induction, expression and purification of a recombinant protein having approximately 31 kDa was achieved. This protein is recognized by the monoclonal antibody mAbAdh, and by the immune serum of hamsters immunized with this protein in Western blot tests, as shown in figure 17. The immunization experiment with the recombinant protein showed that this protein is highly immunogenic, a result which was evidenced by ELISA, showing an IgG specific seric response statistically significant (p .0000001) as regards the control group. A possible booster effect could also be appreciated upon challenging the immunized animals with the protein, since the titter of the specific antibodies was rapidly increased. It was found that this response was also statistically different from that of the control group (p .0000001) and from the response obtained from the same group before the challenge (p .000002). These results are depicted in the graphic appearing on figure 18. Further, 32.5% of protection against hepatic abscesses by amoebas was obtained. These results indicate the possibility of obtaining protection against amoebian infection using formulations containing a fragment of this protein.

Advantages of the solution proposed

This invention has the advantage that it uses as immunogen a surface protein of *Entamoeba histolytica,* which has both adhesin and cystein protease activity, enabling us to fight two key virulence factors of this protozoan parasite. The formulations using these proteins, their constituent sub-units or the genes coding the same enable us to obtain both a specific humoral and cellular response in the host. Since DNA formulations can be used, the advantages of this kind of vaccines are incorporated, such as the relative simplicity in manufacturing and avoiding the need of cold-chains for their transportation, which facilitates a wide coverage, especially in those areas where the disease is endemic.

### Brief description of the figures

**Figure 1.** Electrophoresis in 0.8% agarose gel of the products obtained from the enzymatic analysis of plasmid *pcDNA-Ehadh112.* Lanes: 1) Molecular size marker DNA λ / Hind III, 2) linearised plasmid with digestion by ApaI enzyme, 3) plasmid subjected to double digestion with ApaI and XbaI enzymes, 4) pcDNA₃ vector used for cloning, previously digested with ApaI and XbaI, 5) fragment of adhesin gene used for cloning, 6) Molecular size marker IV (Boeringher).
**Figure 2.** Electrophoresis in 0.8% agarose gel of the products obtained from the enzymatic analysis of plasmid pcDNA-*Ehadh112.* Lanes: 1) Molecular size marker DNA λ / Hind III, 2) linearised plasmid by digestion with BamHI enzyme, 3) plasmid subjected to double digestion with BamHI and EcoRI enzymes, 4) pcDNA₃ vector used for cloning, previously digested with BamHI and EcoRI, 5) cystein protease gene used for cloning, 6) Molecular size marker IV (Boeringher).
**Figure 3**. Electrophoresis in acrylamide gel of RT-PCR products, starting from whole RNA of transfected L929 cells. Lanes: 1) molecular size marker DNA λ / Hind III, 2) product of RT-PCR on RNA isolated from L929 cells transfected with *pCDNA*_{*3*} and amplified with oligonucleotides specific for *Ehadh112* gene, 3) product of RT-PCR on RNA isolated from L929 cells transfected with *pCDNA-Ehadh112* and amplified with oligonucleotides specific for *Ehadh112* gene.
**Figure 4.** Immunolocalization of amoebian proteins Ehadh112 and Ehcp112, in fibroblasts transfected with plasmids *pcDNA-Ehadh112* and *pcDNA-Ehcp112* respectively. Panel A. Transmission image of culture transfected with *pcDNA*_{*3*} (control). Panel B. Culture transfected with *pcDNA*_{*3*} and incubated with the first and the second antibody (control). Panel C. Culture transfected with *pcDNA-Ehadh112* and incubated only with the fluorescein conjugate (control). Panel D. Culture transfected with *pcDNA-Ehadh112* and incubated with both antibodies. Panel E. Culture transfected with *pcDNA-Ehcp112* and incubated with both antibodies.
**Figure 5.** 112 kDa adhesin recognition in whole extracts of amoebian proteins by sera of hamsters immunized with pcDNA-Ehadh112. Lanes: 1) mAb112; 2) pre-immune serum; 3 and 4) serum of hamster administered pcDNA_{3;} 5-8) immune serum of hamsters administered pcDNA-Ehadh112; 9) molecular weight marker (Gibco, USA).
**Figure 6.** Immunodetection of an amoebian surface protein by serum of hamsters immunized with *pcDNA-Ehadh112.* Panel A, trophozoites incubated with a pool of sera from animals administered 2 doses of 100 µg each of pcDNA3 vector (control sera). Panel B, trophozoites incubated with a pool of sera from animals administered 2 doses of plasmid *pcDNA-Ehadh112.*
**Figure 7.** 112 kDa adhesin recognition in whole extracts of amoebian proteins by serum of hamsters immunized with pcDNA-Ehcp112. Lanes: 1) Recognition of a 120 kDa protein; 2) Pre-immune sera; 3, 4 and 5) immune sera from hamsters administered pcDNA-EhCp112; 6) molecular weight marker (Gibco, USA).
**Figure 8.** 112 kDa adhesin recognition in whole extracts of amoebian proteins by sera of hamsters immunized with a mixture of plasmids *pcDNA-Ehadh112* and *pcDNA-Ehcp112.* Lanes: 1) Pre-immune sera; 2) sera from hamsters immunized with the mixture of plasmids *pcDNA-Ehadh112 and pcDNA-Ehcp112*; 3) Recognition of a 120 kDa protein by mAb112, positive control.
**Figure 9.** Kinetics of immune response of anti-amoebian seric IgG antibodies, induced in hamsters immunized with plasmid *pcDNA-Ehadh112.* Values are OD₄₀₅ mean values obtained by sera (1:500) of animals belonging to the same dose group (n=8).
**Figure 10**. Kinetics of immune response of anti-amoebian seric IgG antibodies, induced in hamsters immunized with plasmid *pcDNA-Ehcp112.* Values are OD₄₀₅ mean values obtained by sera (1:500) of animals belonging to the same dose group (n=8).
**Figure 11**. Kinetics of immune response of anti-amoebian seric IgG antibodies, induced in hamsters immunized with a mixture of plasmids *pcDNA-Ehadh112* and *pcDNA-Ehcp112.* Values are OD₄₀₅ mean values obtained by sera (1:500) of animals belonging to the same dose group (n=8).
**Figure 12.** Proliferative response of splenic cells from hamsters vaccinated with *pcDNA-Ehadh112,* to stimulation with amoebian trophozoites. The values represent the mean value of CPM reached by cells of hamsters belonging to the same dose group (n=8).
**Figure 13.** Proliferative response of splenic cells from hamsters vaccinated with *pcDNA-Ehcp112,* to stimulation with amoebian trophozoites. The values represent the mean value of CPM reached by cells of hamsters belonging to the same dose group (n=8).
**Figure 14.** Proliferative response of splenic cells from hamsters vaccinated with a mixture of plasmids *pcDNA-Ehadh112* and *pcDNA-Ehcp112* to stimulation with amoebian trophozoites. The values represent the mean value of CPM reached by cells of hamsters belonging to the same dose group (n=8).
**Figure 15.** Comparison of the inhibitory effect of immune sera upon amoebian adherence to human erythrocytes.
**Figure 16**. Comparison of the protective capacity of immunizations with naked DNA among the groups under study.
**Figure 17.** Recognition of the 31 kDa recombinant polypeptide of the 112 kDa adhesin by sera of immunized hamsters. 1) Pre-immune serum, 2) immune serum of hamsters that received *pcDNA-Ehadh112,* 3) mAb112.
**Figure 18.** Humoral immune response of hamsters immunized with Ehadh240 recombinant protein.

## Claims

1. Vaccinal compositions for the control of amoebiasis, **characterized in that** they comprise as active principle a 112 kDa surface protein of *Entamoeba histolytica* obtained by natural means, whether recombinant or synthetic, peptides derived from the same, as well as the genes coding the same or fragments thereof and an appropriate excipient.

2. A vaccinal composition according to claim 1, **characterized in that** it comprises as active principle the following:
a) a subunit with cystein protease activity coded by an open reading frame of 1338 base pairs (ID. AAF04255.1),
b) a subunit with adhesin activity coded by an open reading frame of 2061 base pairs (ID. AAF04256.1), and
c) a vector appropriate for immunization with DNA.

3. A vaccinal composition according to claim 2 **characterized in that** these subunits are found separately in a concentration range of between 30 µg and 1 mg of proteins.

4. A vaccinal composition according to claim 2 **characterized in that** it comprises a mixture of these subunits in equal proportions and in a range of between 10 µg and 1000 µg.

5. A vaccinal composition according to claim 1 **characterized in that** it comprises as active principle a mixture of peptides, in equal proportions and in a range of between 0.1 mg-1 mg of the peptide.

6. A vaccinal composition according to claim 2 **characterized in that** it comprises one or more appropriate vectors for immunization with DNA, and one of them contains the gene having 1338 base pairs (ID. AAF04255.1) corresponding to the protein with cystein protease activity or the gene having 2061 base pairs (ID. AAF04256.1) corresponding to the protein with adhesin activity at a concentration range of between 5 µg and 800 µg of DNA.

7. Vaccinal compositions according to claim 6 **characterized in that** they comprise a mixture of vectors appropriate for immunization with DNA, wherein the genetic constructions are in equal proportion and at a range of between 10 µg and 800 µg of DNA.

8. Vaccinal compositions according to claim 7 **characterized in that** one of the vectors comprises a fragment of DNA derived from the 1338 base pair gene (ID. SEC. No. 1) corresponding to the protein with cystein protease activity and the other vector has any fragment of DNA derived from the 2061 base pair gene (ID. AAF04256.1) corresponding to the protein with adhesin activity.

9. A vaccinal composition according to claim 8 **characterized in that** the vectors are in equal proportions and at a concentration range of between 30 µg and 1000 µg of the vector.

10. A vaccinal composition according to claim 1 **characterized in that** it comprises one or more appropriate vectors for immunization with DNA, and one of them contains the gene having 1338 base pairs (ID. AAF04255.1) corresponding to the protein with cystein protease activity or the gene having 2061 base pairs (ID. AAF04256.1) corresponding to the protein with adhesin activity, separated by a non-coding gene sequence having 188 base pairs.

11. A vaccinal composition according to claim 10 **characterized in that** the vector is in a rage of between 5 µg and 800 µg.

12. A vaccinal composition according to claim 1 **characterized in that** it comprises as active principle an attenuated live vector expressing the product of the gene having 1338 base pairs (ID. AAF04255.1) corresponding to the protein with cystein protease activity and the product of the gene having 2061 base pairs (ID. AAF04256.1) corresponding to the protein with adhesin activity or fragments of the same.

13. A vaccinal composition according to claim 12 **characterized in that** the attenuated live vector is in a rage of between 20 µg and 100 µg per dose.

14. A vaccinal composition according to claim 13 **characterized in that** the live vector is selected among the group comprising *Mycobacterium bovis,* BCG, *Salmonella sp, Vaccinia,* Adenovirus, Poliovirus, lactic acid bacteria and yeasts.

15. Vaccinal compositions according to claims 1 through 14 **characterized in that** they are administered by any of the routes selected from the group comprising oral, intranasal, vaginal, rectal, intramuscular, subcutaneous and intradermal routes.

16. The use of the vaccinal compositions of claims 1 through 15 for the prophylaxis or treatment of the infection caused by *Entamoeba histolytica.*
